Europäisches Patentamt

(19) European Patent Office

Office européen des brevets

(11) EP 0 855 190 A1

(12) EUROPEAN PATENT APPLICATION

(43) Date of publication:
29.07.1998 Bulletin 1998/31

(51) Int. Cl.$^6$: **A61L 15/58**, C09J 153/00, C09J 153/02

(21) Application number: 97120338.5

(22) Date of filing: 20.11.1997

(84) Designated Contracting States:
AT BE CH DE DK ES FI FR GB GR IE IT LI LU MC NL PT SE
Designated Extension States:
AL LT LV MK RO SI

(30) Priority: 23.12.1996 EP 96120738
01.07.1997 EP 97110730

(71) Applicant:
THE PROCTER & GAMBLE COMPANY
Cincinnati, Ohio 45202 (US)

(72) Inventors:
• Corzani, Italo
66100 Chieti (IT)
• Romano, Mario
65125 Pescara (IT)

(74) Representative:
Hirsch, Uwe Thomas et al
Procter & Gamble European Service GmbH,
Sulzbacher Strasse 40-50
65824 Schwalbach am Taunus (DE)

(54) **Adhesive for secure topical attachment to the skin and comfortable removal, particularly of absorbent articles**

(57) The present invention relates to topical adhesives for attachment to the skin. In particular the present invention relates to such topical adhesives which can be employed for attachment to the skin in the area were absorption of bodily liquids is desired. The topical adhesive provides secure attachment and is pleasing to the skin upon application, yet causes no discomfort upon removal. This is achieved by selecting the chemical composition and rheological characteristics of the topical adhesives.

EP 0 855 190 A1

Printed by Xerox (UK) Business Services
2.16.3/3.4

**Description**

Field of the invention

The present invention relates to topical adhesives for attachment to the skin. In particular the present invention relates to such topical adhesives which can be employed for attachment to the skin in the area were absorption of bodily liquids is desired, particularly for the adhesion of sanitary napkins, pantiliners, adult incontinence products or sweat pads. The topical adhesive provides secure attachment and is pleasing to the skin upon application, yet causes no discomfort upon removal. This is achieved by selecting the chemical composition and rheological characteristics of the topical adhesives.

Background of the invention

The general prior art in the field of topical adhesives for attachment to the skin is particularly developed in the field of band-aids, plasters and bandages. These articles are, however, typically applied in an emergency situation where for example a cut into the skin of the wearer has occurred and absorption of the body liquids emanating from a wound is desired. In this context performance aspects of the absorbent article such as comfortable and easy use and application, painless removal, discreteness are subordinate to criteria such as sterility, healing support, mechanical protection of the wound. Also such wound covering absorbent articles are mostly adhered to skin areas where prior to application of the absorbent article body hair can be removed or where little or no hair grows.

The present invention relates to topical adhesives which are particularly useful to absorbent articles for absorption of body liquids which naturally emanate from a body without a wound. For example to attach sanitary napkins or pantiliners in the genital region. Also incontinence devices which are worn e.g. in the genital region or sweat pads which are worn in the arm pit region of a person can suitably employ the adhesive of the present invention.

Such topical adhesives have generally been disclosed in US statutory invention registration H1602 or WO 96/33683. Some more details of the adhesive have been disclosed in PCT application WO 95/16424. In this document sanitary articles having a topical adhesive which is applied on the wearer facing side of a sanitary napkin along the entire periphery are disclosed. The problem underlying this document is primarily the safe attachment to the skin but mentions also the problems of detachment of such articles after use without causing undue pain to a wearer.

The disclosure of WO 95/16424 includes a detailed analysis of the criteria for the topical adhesive in respect to rheological criteria. However, this document has little regard to the problem of painless removal of such articles since the rheological criteria taught include epilatory, i.e. hair removal, compositions which are commercially available such as STREP MIELE (TM) sold in Italy by Laboratori Vaj S.p.A. The adhesives for topical attachment mentioned in WO 95/16424 include also today's pressure sensitive adhesives which are used to attach sanitary napkins to undergarments. Further, this document only identifies static rheological characteristics but is silent as to the dynamic rheological behaviour of a topical adhesive.

In WO 96/13238 a frequency dependent topical adhesive model is disclosed. However, all measurements disclosed, e.g. on page 9, were made at temperatures between -60°C and +120°C and at actual frequencies of 0.1 to 100 rad/s. In order to obtain the necessary data at application temperature (about 20°C, typical bath room, i.e. storage temperature) the Williams-Landel-Ferry (hereinafter WLF) equation was used.

This WLF equation is empirical and only valid within certain limits e.g. it cannot be used to extrapolate to temperatures below the glass transition temperature of a polymeric adhesive also the WLF cannot be used on the basis of values obtained below the glass transition temperature. Details about the WLF equation and its applicability can be found in "Principles of Polymer processing" by Z. Tadmor and C.G. Gogos, published by John Wiley & Sons or in "Viscoelastic Properties of Polymers" by J.D. Ferry also published by John Wiley & Son. Since this is already missing from WO 96/13238 the applicability of the disclosed data cannot be assessed.

European Patent Application EP-638 303 discloses the use of a topical adhesive on side cuffs of sanitary napkins in order to keep the cuffs in an upright position. Swiss publication CH-643730 discloses the use of a very long sanitary napkin having chamfered outer edges with a topical adhesive at the four corners of the outer edges in order to provide a topical adhesive area well outside the region of pubic hair growth. Both applications are silent as to the adhesive composition.

Based on the above state of the art it is an objective of the present invention to provide a topical adhesive for secure attachment and painless removal from the skin. It is another objective of the present invention to ensure upon removal that no residual adhesive remains on the skin or on the hair.

It is yet a futher objective of the present invention that the adhesive for topical attachment does not cause a cold or otherwise unacceptable temperature sensation upon application despite a temperature difference of the adhesive in respect to the skin temperature.

In addition to the above objectives of the present invention it is also desirable for topical adhesives to provide additional benefits such as delivery/ dispersal of a compound or composition which is beneficial for the skin or for the body in general. Further, topical adhesives which do not affect the natural skin condition/ e.g. by being breathable or water vapour transmitting or water vapour/ sweat absorbing, are preferred.

Brief description of the invention

The present invention will be explained in its use on disposable absorbent articles for topical adhesive attachment to a wearer of such articles. The article typically has a wearer facing surface and an outside surface. The article comprises an absorbent core structure between the wearer facing surface and the outside surface for absorbing liquids emanating from a wearer such as urn, feces, menses, sweat and vaginal discharge. The topical adhesive allows to attach an article to the skin of the wearer.

Detailed analysis of the sequence of common situations occurring from the application of a disposable absorbent article to the time of removal of such an article has shown that specific adhesive characteristics need to be satisfied in order to achieve the desired performance objectives, in particular secure initial attachment , secure attachment during use and painless removal at the end. The characteristics which have been considered in this context are the elastic modulus describing the elastic behaviour of the material and the viscous modulus which describes the viscous behaviour of the adhesive material.

The viscous behaviour of the adhesive can be interpreted to represent an indication of the ability of the adhesive to quickly attach and securely adhere. The elastic behaviour can be interpreted as an indication of the "hardness" behaviour of the adhesive. Its value is also critical for good initial attachment. Their combination is believed to be an indicator of the required force upon removal. The relation between elastic and viscous modulus is considered to be an indication which fraction of the removal energy will be dissipated within the adhesive and which fraction is available to trigger the actual removal.

In order to provide topical adhesives for secure initial and prolonged attachment and easy/painless removal the relation between the elastic modulus and the viscous modulus as well as their dynamic behaviour is of key importance.

The topical adhesive has an elastic modulus at a temperature of 37°C (100° Fahrenheit) abbreviated $G'_{37}$ and a viscous modulus at a temperature of 37°C (100° Fahrenheit) of $G''_{37}$. The adhesive further has a dynamic elastic behaviour defined as $\Delta G'_{37}$ which is the difference of $G'_{37}$ at a frequency of 100 rad/sec and $G'_{37}$ at a frequency of 1 rad/sec and a dynamic viscous behaviour $\Delta G''_{37}$ which is the difference of $G''_{37}$ at a frequency of 100 rad/sec and $G''_{37}$ at a frequency of 1 rad/sec.

The topical adhesive according to the present invention satisfies the following conditions.

• $G'_{37}$ (1 rad/sec)    is in the range 1500 Pa to 20000 Pa, preferably 1500 Pa to 15000 Pa, most preferably 3000 Pa to 10000 Pa.

• $G''_{37}$ (1 rad/sec)    is in the range 100 Pa to 15000 Pa, preferably 100 Pa to 10000 Pa, most preferably 300 Pa to 5000 Pa.

• the ratio of    $G'_{37}$ (1 rad/sec) / $G''_{37}$ (1 rad/sec) is in the range of 3 to 30.

• the ratio

$$\frac{G'_{37} \ (100 \ rad/sec) - G''_{37} \ (100 \ rad/sec)}{G'_{37} \ (1 \ rad/sec) \ - \ G''_{37} \ (1 \ rad/sec)}$$

is not less than 0.5, preferably in the range 0.7 to 3, most preferably in the range 1 to 1.8.

• either the ratio of    $\Delta G'_{37}/G'_{37}$ (1 rad/sec) is not greater than 1.5, preferably not greater than unity and most preferably not greater than 0.8,
or $\Delta G'_{37}$ is not greater than 10000 Pa, preferably less than 5000 Pa, most preferably less than 2000 Pa,
or both.

• the value of the ratio $G'_{37}/G''_{37}$ at least for the frequency range from above 1 rad/s up to 100 rad/s should preferably be 3.3 or above more preferably 5 or above, most preferebly 10 or above, while not exceeding about 50, preferably 30, anywhere in the frequency interval.

- the rheological behaviour can also be related to the values of the Glass Transition Temperature Tg. For topical adhesives according to the present invention Tg should preferably be less than -15°C, more preferably less than -20°C and most preferably less than -25°C.

- the rheological behaviour and acceptance of a topical adhesive can also be related to the specific heat capacity. Preferably the specific heat capacity of the topical adhesive is less than 4 J/g/K, more preferably less than 3 J/g/K and most preferably less than 2 J/g/K.

- the rheological behaviour and acceptance of a topical adhesive can also be related to the specific heat conductivity of the adhesive. Preferably the specific heat conductivity is as low as possible, preferably between 1 and 0.1 W/m/K, most preferably between 0.6 and 0.1 W/m/K.

Provided the above rheological conditions are satisfied the adhesives will also satisfy conditions such as sufficient cohesiveness (to prevent residue of adhesive on skin) which are critical for commercial use of such adhesives and apparent to those skilled in the art. Adhesive compositions which satisfy the above criteria can be used as topical adhesives for disposable absorbent articles provided they also satisfy the common requirements of being safe for use on human or animal skin during use and generally after disposal of the article.

Often the criteria of hygienic appearance and pleasant feel upon contact are important such that adhesive composition which are transparent or white, and which prevent a cold, unpleasant feeling upon application are preferred.

The above rheological criteria and other considerations can be satisfied by adhesive compositions where the composition comprises from 45%, preferably from 51 %, to 99.5 % of a plasticising compound or composition which is liquid at 20°C, from 0.5 to 20 %, preferably 5 % to 15 %, of a polymeric compound or composition which is soluble or swellable in the plasticising compound or composition and with a tackifying resin in an amount in the range from 0% to 50% by weight of the composition, prefereably in the range from 0 % to 600 % by weight of the polymeric compound. The plasticising compound or composition is preferably selected from the group consisting of water, alcohols (preferably glycerol), glycols, polyglycols, liquid polybutenes, oil or combinations thereof. The polymeric compound or composition is preferably selected from the group consisting of block-copolymer-thermoplastic-elastomers, styrene-blockcopolymers and hydrogenated styrene-block-copolymers.

Detailed description of the invention

Adhesive for topical attachment

The topical adhesive according to the present invention is applied directly to the skin. In a particular application the adhesive can be used on sanitary napkins which are applied in the genital region of a typically female user around the area of liquid discharge. The word "skin" according to the present invention does not only relate to the specific derma of the user but includes the mucous tissue as well as the hair which is typically found in the genital region of users of sanitary napkins.

In order to provide fixation of an article according to the present invention to the skin it is necessary to provide a certain area on the side of the article which is facing the skin with the topical adhesive.

For sanitary napkins various designs are contemplated but preferably the topical adhesive is provided along the peripheral edge of the topsheet such that a central area of the article is left without adhesive. This will most appropriately facilitate placing the napkin such that the liquid permeable topsheet region without adhesive is placed adjacent the bodily liquid emanating orifice such that emanating liquid is immediately transported into the absorbent structure of the absorbent article without the possibility of leakage or spillage.

Physical, Rheological and Adhesive Characteristics of a Topical Adhesive

Even so topical adhesives are used like pressure sensitive adhesives on human skin hair and mucous tissues, it is understood that the topical adhesive compositions could only with difficulty be considered typical pressure sensitive adhesives (referred to as PSA hereinafter) on the basis of the most characteristic rheological behaviours identifying such materials.

In fact as the person skilled in the art of adhesives knows, the most characteristic feature that distinguish a PSA from other substances that can temporarily stick things (as e.g. water between two glass plates could) is the fact that their rheological parameters and especially the Elastic Modulus G' vary greatly with the frequency of applied stresses.

More in particular, G' of PSA can increase over some orders of magnitude while the frequency of applied stresses varies from typical bonding frequency to typical debonding frequency, i.e. 1 rad/s to 100 rad/s as indicated below.

As a first consequence, it derives that it is inadmissible to define materials intended for use as " topical adhesives" by giving values of rheological parameters and especially of G' at a fixed value of frequency. This can be misleading because in the absence of other characteristics it will include materials which have no practical value. It is hence necessary that rheological characterisation must be on the base of dynamic considerations.

This not only applies to the Elastic Modulus G' but also to the viscous modulus G" and hence also for tan $(\delta)$ = G" / G'. It is well known that typical PSA have not only a high variation of G' across the considered frequencies but also there is an even higher variation of G" which can get close or become even higher than the value of G', i.e. tan $(\delta)$ becomes about or even greater than 1, in particular at the frequencies that are typical of the debonding.

Without wishing to be bound by theory this can be interpreted as meaning that a high fraction of the energy applied for the debonding is dissipated within the adhesive (so it is not effective in causing the debonding) while this fact causes macroscopically the recording of a very high level of adhesive force.

As indicated above materials useful as topical adhesives according to the present invention have rheological characteristics which are measured at a reference temperature of 37°C (as usual body temperature of humans) and in a range of frequencies. It has been found that upon application of an article such as a sanitary napkin with a topical adhesive the adhesive contact is formed at a low frequency, while debonding happens at the speed of removing the article. This speed is expressed as a frequency of 100 rad/s while the low frequency of forming the adhesive bond has been found to be on the order of 1 rad/s. Therefore, the frequency range for use according to the present invention is between 1 and 100 rad/s.

It is believed that the adhesive bonding characteristics are selected most appropriately at human body temperature. Since the topical adhesive according to the present invention is used directly on skin and the person skilled in the art is directed to select the adhesive composition to have a small specific heat capacity (e.g. preferably less than 4 J/g/K) the actual temperature of the topical adhesive will reach 37°C very quickly or even be warmed up by a human prior to application.

In order to provide good conditions of bonding, i.e. at a frequency of about 1 rad/sec, the absolute values of the elastic modulus should not be too high, otherwise the adhesive is too hard and it is not able to intimately join or mold to the surface to which it is expected to adhere. It is also important to have a low absolute value of G" in order to have good cohesion which is particularly valuable for use in the field of sanitary napkins while the material remains soft and capable of gently adhering to skin.

The ratio of $G'_{37}$ (1 rad/sec) over $G''_{37}$ (1 rad/sec) is important to ensure that these two values are balanced upon adhesion to the skin. At the same time the absolute changes of $G'_{37}$ needs to be limited within the range of frequencies considered. Hence a value for the ratio of $\Delta G'_{37}$ (i.e. $G'_{37}$ (100 rad/sec) - $G'_{37}$ (1 rad/sec)) over $G'_{37}$ (1 rad/sec) has to be kept small in order to maintain the secure attachment of the topical adhesive without causing discomfort over time or at removal/ delamination. This can also be expressed in absolute terms by keeping the $\Delta G'_{37}$ below certain values.

Importantly, the ratio of

$$\frac{G'_{37} \ (100 \ \text{rad/sec}) - G''_{37} \ (100 \ \text{rad/sec})}{G'_{37} \ (1 \ \text{rad/sec}) \quad - \quad G''_{37} \ (1 \ \text{rad/sec})}$$

needs to be large enough to ensure that the dynamic behaviour of both the elastic and the viscous module are maintained in a relationship which provides secure adhesion and painless and easy removal.

Finally the person skilled in the art will also recognise that the Glass Transition Temperature Tg of the adhesive composition, specific heat capacity, and specific heat conductivity are parameters which are useful to more fully define the group of useful topical adhesives.

The following set of characteristics should be satisfied:

• $G'_{37}$ (1 rad/sec)      is in the range 1500 Pa to 20000 Pa, preferably 1500 Pa to 15000 Pa, most preferably 3000 Pa to 10000 Pa.

• $G''_{37}$ (1 rad/sec)      is in the range 100 Pa to 15000 Pa, preferably 100 Pa to 10000 Pa, most preferably 300 Pa to 5000 Pa.

• the ratio of      $G'_{37}$ (1 rad/sec) / $G''_{37}$ (1 rad/sec) is in the range of 3 to 30.

• the ratio

$$\frac{G'_{37} \text{ (100 rad/sec)} - G''_{37} \text{ (100 rad/sec)}}{G'_{37} \text{ (1 rad/sec)} \quad - \quad G''_{37} \text{ (1 rad/sec)}}$$

is not less than 0.5, preferably in the range 0.7 to 3, most preferably in the range 1 to 1.8.

• either the ratio of $\Delta G'_{37}/G'_{37}$ (1 rad/sec) is not greater than 1.5, preferably not greater than unity and most preferably not greater than 0.8,
or $\Delta G'_{37}$ is not greater than 10000 Pa, preferably less than 5000 Pa, most preferably less than 2000 Pa,
or both.

• the value of the ratio $G'_{37}/G''_{37}$ at least for the frequency range from above 1 rad/s up to 100 rad/s should preferably be 3.3 or above more preferably 5 or above, most preferably 10 or above, while not exceeding about 50, preferably 30, anywhere in the frequency interval.

• the rheological behaviour can also be related to the values of the Glass Transition Temperature Tg. For topical adhesives according to the present invention Tg should preferably be less than -15°C, more preferably less than -20°C and most preferably less than -25°C.

• the rheological behaviour and acceptance of a topical adhesive can also be related to the specific heat capacity. Preferably the specific heat capacity of the topical adhesive is less than 4 J/g/K, more preferably less than 3 J/g/K and most preferably less than 2 J/g/K.

• the rheological behaviour and acceptance of a topical adhesive can also be related to the specific heat conductivity of the adhesive. Preferably the specific heat conductivity is as low as possible, more preferable between 1 and 0.1 W/m/K, most preferably between 0.6 and 0.1 W/m/K.

Chemical and compositional characteristics of a Topical adhesive

In order to provide topical adhesive compositions which satisfy the requirements of the above rheological and physical characteristics of a topical adhesive the following formulation criteria can be used in addition. It should be noted that the most of the compositions useful as topical adhesive have a substantially gel-like structure and are preferably gels. This derives from the fact that:

- the prevailing component is the plasticiser which is a material liquid at room temperature
- a macromolecular or polymeric component is present in minor quantities vs the plasticiser. It forms, in the preferred embodiments, a three dimensional network caused by physical or chemical links between the molecules. Particularly useful physical links are the ones present in systems containing Block Thermoplastic Elastomers.

More specifically, the compositions typically comprise:

- from 0.5 to 20 %, preferably 5 % to 15 %, by weight of a macromolecular polymeric substance or a mixture of such substances soluble or swellable in the below mentioned plasticiser(s). As not limiting examples such macromolecular or polymeric substances can be natural and/or synthetic such as natural gums or derivatives such as natural gums and gelatines, their derivatives and alginates; polyacrilics; polyvinyl alcohol; polyethylene oxide; polyvinylpyrrolidon (PVP) or polyvinylethers, their copolymers and derivatives; cellulose derivatives; Block Copolymer Thermoplastic Elastomers and preferably Styrenic Block Copolymers and more preferably the hydrogenated grades Styrol/Ethylene-Butylene/Styrol (SEBS), Styrene/Isoprene/Styrene (SIS), and Styrol/Ethylene-Propylene/Styrol (SEPS).

- from 45 to 99.5 % by weight, preferably from 51 to 99.5% by weight, of a plasticising substance or a mixture of plasticising substances, which are liquid at room temperature. As non-limiting examples the plasticiser can be water, various alcohols (like in particular glycerol), glycols and their eithers, polyglycols, liquid polybutenes, esters such phtalates, adipates, stearates, palmitates, sebacates, or myristates, natural or synthetic oils such as vegetable oils, mineral oils, or combinations thereof.

- from 0% to 50% by weight, preferably from 0% to 600 % by weight of the macromolecular polymeric substance, of a tackifying resin whose main scope is to tailor the Tg especially in systems based on synthetic polymers.

- from 0 to 10 % and more preferably form 0 to 5 % by weight of substances for facilitating and stabilising the gel and the gel forming process both of hydrophilic or hydrophobic liquid plasticisers. These may be for oily systems, e.g. the fatty acids of $C_8$ to $C_{22}$, their metallic salts and their polyoxo-derivatives; lanolin derivatives; silica; bentonite, montmorillonite and their derivatives; polyamides, waxes or mixtures thereof.

Common additives known in the art as preservatives, antioxidants, anti UV, pigments, mineral fillers, rheology modifiers etc. can also be comprised in quantities up to 10 % each.

When chemical crosslinks are formed in the system, a crosslinking agent can be present preferably in quantities up to 5 % by weight. Chemical crosslinking can be formed also by mutual neutralisation of polymers having different functionalities as in the reaction between acid polyacrylics and polysaccharides.

The resulting compositions for topical adhesives can be divided into three families according to the nature of the main component, i.e. usually the liquid plasticiser(s):

1) Hydrophobic compositions in which the plasticiser is typically an oil or blend of oils of vegetable or mineral origin and the polymer is usually a synthetic polymer, preferably an elastomer, soluble or swellable in oil(s).

2) Mixed phase compositions in which both hydrophobic and hydrophilic components, possibly in both plasticisers and polymers, form two or more separate phases. In such cases an emulsifier/surfactant is preferably present at a suitable level to form stable emulsions between the incompatible phases. For topical adhesives according to the present invention it is preferably that the hydrophobic components are prevailing vs. the hydrophilic ones.

3) Hydrophilic compositions in which typically the plasticiser is water/glycerol/glycols and the like and/or mixtures thereof and the polymeric phase is of synthetic (e.g. polyacrilics) or natural (e.g. natural gums) origin or mixtures thereof.

It is to stress that, differently from the medical field and from the cited prior art, the hydrophilic compositions are not preferred while the hydrophobic and mixed phases compositions 1) and 2) are preferred in the applications of the present invention.

This depends partially on technical reasons in the sense that many hydrophilic compositions used in the medical field show too low elastic character and cohesion for being useful in the present application. The other reason to prefer hydrophobic or mixed phase compositions is that the application of the present invention in particular in the sanitary napkin field will include a probability of contacting the topical adhesive with the liquid to be absorbed. Since the liquids are all of a general aqueous kind contact with a hydrophilic topical adhesive would result in a certain absorption of the bodily liquids into the topical adhesives.

This would then have the result of changing the rheological characteristics and therefore the functionality of the topical adhesive and causing a nonhygienic appearance.

Further hydrophilic topical adhesive also tend to be perceived as cold and wet which upon application to the skin of a human is not in line with typical expectation. Additional problems result from the fact that in particular topical adhesives comprising water as the plasticiser have a tendency to dry out unless they are sealed into an impermeable package.

Application of topical adhesive

Absorbent articles in which the topical adhesive according to the present invention can be used, can be made by any of the ways usual in the art. The application of the adhesive to the topsheet side of an absorbent article should not cause major problems to those skilled in the art since it can be provided by any well known techniques commonly used for other adhesives. The total area of the wearer facing surface of an absorbent article which is covered by the topical adhesive should be not more than 20 %, preferably not more than 10 %. Preferably, the adhesive is close to the periphery of the absorbent article and in the case of film topsheets (or when the backsheet is folded onto the topsheet) the adhesive is preferably on a portion of the film which is not permeable to liquids.

The topical adhesive on an article is preferably protected prior to use. This protection can be provided by a release liner such as a siliconised or surfactant treated paper, providing easy release for the selected topical adhesive.

This invention can be used beneficially on disposable absorbent articles which are applied directly to the skin of a user. The article usually exhibits absorbency for bodily fluids, the protection of the user's garments from soiling, is comfortable to the user, and is easy to produce and to package. The disposable absorbent article is described below by reference to a sanitary napkin or catamenial, however panty liners, adult incontinence articles or sweat pads are also included under the term disposable absorbent articles. The term "sanitary napkin", as used herein, refers to an article which is worn by females adjacent to the pudendal region and which is intended to absorb and contain the various body fluids which are discharged from the body (e.g., vaginal discharges, menses, and/or urine) and which is intended to be discarded after a single use. A disposable absorbent article is preferably thin, more preferably between 1 and 5 mm thick and either substantially flat prior to use or in a preshaped form.

The terms "joined" or "affixed", as used herein, encompasses configurations whereby a first member is directly connected to a second member and configurations whereby a first member is indirectly connected to a second member by connecting the first member to intermediate members which in turn are connected to the second member.

In a preferred embodiment a sanitary napkin of the present invention comprises a liquid pervious topsheet, a liquid impervious backsheet joined to the topsheet, and an absorbent core intermediate the topsheet and the backsheet. The sanitary napkin has two main surfaces, a body contacting or wearer facing surface on which the topical adhesive is applied and a garment facing or contacting surface.

The topsheet is compliant, soft feeling, and non-irritating to the wearer's skin. The topsheet also can have elastic characteristics allowing it to be stretched in one or two directions in portions of the topsheet or throughout its extension. Further, the topsheet is fluid pervious permitting fluids (e.g., menses and/or urine) to readily penetrate through its thickness.

Preferred topsheets for use in the present invention are typically selected from high loft nonwoven topsheets and apertured formed film topsheets. Apertured formed films are especially preferred for the topsheets because they are pervious to body exudates and yet non absorbent and have a reduced tendency to allow fluids to pass back through and rewet the wearer's skin. Thus, the surface of the formed film that is in contact with the wearer remains dry, thereby reducing body soiling and creating a more comfortable feel for the wearer. Suitable formed films are described in U.S. Patent 3,929,135; U.S. Patent 4,324,246; U.S. Patent 4,342,314; U.S. Patent 4,463,045; and U.S. Patent 5,006,394. Particularly preferred micro apertured formed film topsheets are disclosed in U.S. patent 4,609,518 and U.S. patent 4,629,643. A preferred topsheet for the present invention comprises the formed film described in one or more of the above patents and marketed on sanitary napkins by The Procter & Gamble Company of Cincinnati, Ohio as "DRI-WEAVE".

Topical adhesives are most suitably used on topsheets having not a homogeneous distribution of liquid passage ways but only a portion of the topsheet comprising liquid passage ways oriented such that they result in a centrally permeable and peripherally impermeable topsheet for liquids.

Another alternative are so called hybrid topsheets which incorporate fibrous and film like structures particularly useful embodiments of such hybrid topsheets are disclosed in PCT publications WO 93/09744; WO 93/11725 or WO 93/11726.

When referring to the topsheet a multi layer structure or a mono layer structure is contemplated. The hybrid topsheet mentioned above is such a multi layer design but other multi layer topsheets such as primary and secondary topsheet designs are also considered.

The absorbent core also can comprise multiple layers and provides fluid storage and distribution function.

Positioned in fluid communication with, and typically underlying the topsheet is the absorbent core. The core can comprise any usual absorbent material or combinations thereof. It preferably comprises absorbent gelling materials usually referred to as "hydrogel", "superabsorbent", hydrocolloid" materials in combination with suitable carriers.

Suitable absorbent gelling materials for use herein will most often comprise a substantially water-insoluble, slightly cross-linked, partially neutralised, polymeric gelling material. This material forms a hydrogel upon contact with water. Such polymer materials can be prepared form polymerizable, unsaturated, acid-containing monomers, such as acrylic acid, which are well known in the art.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins/panty liners.

An embodiment of the core, particularly useful in the application of the present invention, comprises a double layer tissue laminate formed by folding the tissue onto itself. These layers can be joined to each other. Absorbent gelling material or other optional material can be c omprised between the layers.

The absorbent core can include optional components normally present in absorbent webs such as odor control agents, in particular suitable zeolites.

The backsheet primarily prevents the exudates absorbed and contained in the absorbent core from wetting articles that contact the absorbent product such as underpants, pants, pyjamas and undergarments. The backsheet is preferably impervious to liquids (e.g. menses and/or urine) and usually manufactured from a thin plastic film.

The backsheet typically extends across the whole of the absorbent core and can extend onto and form part of the topsheet by folding around the absorbent core. Thereby a topsheet configuration as disclosed in US 4,342,314, column 16, lines 47 - 62 can be achieved without the requirement to selectively aperture the topsheet.

Preferably, the backsheet also provides breathability to the absorbent article by being at least water vapour permeable, preferably air permeable.

The backsheet can be a laminate material e.g. of a combination of microporous film and/or non-woven material, and/or apertured formed film. Breathability if desired can be limited to the periphery or the center of the backsheet or it can be across the whole backsheet.

## EXAMPLE 1

An oil based composition useful on sanitary napkins according to the present invention was prepared using 9.9 % by weight of Kraton G-1651, a Styrene/Ethylene-Butylene/Styrene block copolymer containing 33% by weight styrene and available from Shell Co, and 59.3 % by weight of Kaydol, a paraffinic mineral oil available from Witco Co.

Moreover the composition contained 301 parts of tackifying resin per 100 parts of Kraton polymer. The tackifying resin was Escorez 5300, a hydrogenated resin available from Exxon Co.

Magnesium Stearate, available from Carlo Erba S.p.A., was used a co-gelifying agent for oil at a level of 0.7 % by weight.

Irganox 1010, an antioxidant available from Ciba-Geigy, was added at a level of 0.3 % by weight.

So finally the formulation had the following percent composition:

| | |
|---|---|
| Kraton G-1651 | 9.9 % by weight |
| Kaydol | 59.3 % by weight |
| Escorez 5300 | 29.8 % by weight |
| Magnesium Stearate | 0.7 % by weight |
| Irganox 1010 | 0.3 % by weight |

The composition showed the following rheological properties at 37°C.

a) Elastic Modulus at 1 rad/s, $G'_{37} = 6876$ **Pa**
b) Viscous Modulus at 1 rad/s, $G''_{37} = 550{,}5$ **Pa**
c) Ratio of Elastic and Viscous Modulus at 1 rad/s, $G'_{37} / G''_{37} = 12.49$
d) Ratio of

$$\frac{G'_{37} \ (100 \ rad/sec) - G''_{37} \ (100 \ rad/sec)}{G'_{37} \ (1 \ rad/sec) \quad - \quad G''_{37} \ (1 \ rad/sec)} = 1.22$$

e) The ratio of $\Delta G'_{37}$ over $G'_{37}$ (1 rad/s) was 0.308, with $\Delta G'_{37} = 2124$ Pa.

The above formulation was tried in a sanitary napkin and was judged as comfortable for initial application and removal from sensitive, hairy skin.

## COMPARATIVE EXAMPLE

A comparative oil based composition was compounded using 7.1 % by weight of Kraton G-1651, a Styrene/Ethylene-Butylene/Styrene block copolymer containing 33% by weight styrene and available from Shell Co, and 41.9 % by weight of Kaydol, a paraffinic mineral oil available from Witco Co.

Moreover the composition contained 704 parts of tackifying resin per 100 parts of Kraton polymer. The tackifying resin was Regalrez 3102, a hydrocarbon resin available from Hercules Co.

Magnesium Stearate, available from Carlo Erba S.p.A., was used a co-gelifying agent for oil at a level of 0.7 % by weight.

Irganox 1010, an antioxidant available from Ciba-Geigy, was added at a level of 0.3 % by weight.

So finally the formulation had the following percent composition:

| Kraton G-1651 | 7.1 % by weight |
| Kaydol | 41.9 % by weight |
| Regalrez 3102 | 50.0 % by weight |
| Magnesium Stearate | 0.7 % by weight |
| Irganox 1010 | 0.3 % by weight |

The composition showed the following rheological properties at 37°C.

a) Elastic Modulus at 1 rad/s, **$G'_{37} = 3059\ Pa$**
b) Viscous Modulus at 1 rad/s, **$G''_{37} = 1208\ Pa$**
c) Ratio of Elastic and Viscous Modulus at 1 rad/s, **$G'_{37} / G''_{37} = 2.53$**
d) Ratio of

$$\frac{G'_{37}\,(100\ rad/sec) - G''_{37}\,(100\ rad/sec)}{G'_{37}\,(1\ rad/sec)\ -\ G''_{37}\,(1\ rad/sec)}$$

$$= -2.87$$

e) The ratio $\Delta G'_{37}$ over $G'_{37}$ (1 rad/s) was 3.944 with $\Delta G'_{37} = 12064.7\ Pa$.

The above formulation was judged as highly uncomfortable for delamination from fore-arm skin. Application to sensitive hairy skin was unacceptable.

**Claims**

1. A topical adhesive for attachment of a disposable absorbent article to skin

   - said adhesive having an elastic modulus at a temperature of 37°C (100°F), $G'_{37}$, and having a viscous modulus at a temperature of 37°C (100°F), $G''_{37}$,

   - said adhesive being selected to have

     • $G'_{37}$ (1 rad/sec) in the range 1500 Pa to 20000 Pa, preferably 1500 Pa to 15000 Pa, most preferably 3000 Pa to 10000 Pa;

     • $G''_{37}$ (1 rad/sec) in the range 100 Pa to 15000 Pa, preferably 100 Pa to 10000 Pa, most preferably 300 Pa to 5000 Pa;

     • the ratio $G'_{37}$ (1 rad/sec) / $G''_{37}$ (1 rad/sec) is in the range 3 to 30;

     • the ratio

$$\frac{G'_{37} \text{ (100 rad/sec)} - G''_{37} \text{ (100 rad/sec)}}{G'_{37} \text{ (1 rad/sec)} - G''_{37} \text{ (1 rad/sec)}}$$

is not less than 0.5, preferably in the range 0.7 to 3, most preferably in the range 1 to 1.8;

- alternatively either

  - $G'_{37}$ (100 rad/sec) - G' (1 rad/sec) is not greater than 10000 Pa, preferably less than 5000 Pa, preferably less than 2000 Pa;
    
    or

  - the ratio

$$\frac{G'_{37} \text{ (100 rad/sec)} - G'_{37} \text{ (1 rad/sec)}}{G'_{37} \text{ (1 rad/sec)}}$$

is not greater than 1.5, preferably not greater than 1, most preferably not greater than 0.8, or a combination thereof.

2. A topical adhesive according to claim 1 wherein the value of the ratio $G'_{37}$ over $G''_{37}$ in the frequency range 1 - 100 rad/s is in the range 3.3 to 30.

3. A topical adhesive according to any of the preceding claims comprising

   - from 45 % to 99.5 %, preferably 51 % to 99.5 %, by weight of a plasticising compound or composition which is liquid at 20°C;

   - from 0.5 % to 20 % by weight of a polymeric compound or composition which is solvable or swellable in said plasticising compound or composition;

   - a tackifying resin in an amount of from 0% to 50% by weight, preferably from 0 % to 600 % by weight of said polymeric compound or composition.

4. A topical adhesive according to claim 3 wherein

   - said plasticising compound or composition is selected from the following group: water, alcohols, glycols, oil or combinations thereof; and

   - said polymeric compound or composition is selected from the following group: block-copolymer-thermoplastic-elastomers, styrene-block-copolymers and hydrogenated styrene-blockcopolymers.

5. A topical adhesive according to any of the preceding claims wherein 80 % by weight of said adhesive consist of hydrophobic components and most preferably all components of said adhesive are hydrophobic.

6. A topical adhesive according to any of the preceding claims wherein said adhesive has a glass transition temperature of less than -15°C, preferably less than - 20°C, most preferably - 25°C.

7. A topical adhesive according to any of the preceding claims wherein said adhesive has a specific heat capacity of less than 4 J/g/K, preferably less than 3 J/g/K, most preferably less than 2 J/g/K.

8.  A topical adhesive according to any of the preceding claims wherein said adhesive has a specific heat conductivity of less than 1 W/m/K, preferably in the range of 0.1 to 1 W/m/K, most preferably in the range of 0.1 to 0.6 W/m/K.

9.  Use of a topical adhesive according to any of the preceding claims on a sanitary napkin or panty-liner.

**European Patent Office**

## EUROPEAN SEARCH REPORT

Application Number

EP 97 12 0338

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | US 5 559 165 A (PAUL CHARLES W)<br>* column 1, line 21 - line 35 *<br>* column 1, line 54 - column 2, line 6 *<br>* column 3, line 31 - column 4, line 40 *<br>* column 4, line 64 - line 66 *<br>* claims; examples * | 1,3-5,9 | A61L15/58<br>C09J153/00<br>C09J153/02 |
| Y,D | WO 96 13238 A (KIMBERLY CLARK CO)<br>* page 7, line 11 - page 10, line 6 *<br>* page 2, line 15 - line 35 *<br>* claims; examples 1-4 * | 1-9 | |
| Y,D | WO 95 16424 A (KIMBERLY CLARK CO)<br>* page 16, line 3 - page 18, line 16 * | 1-9 | |
| A | WO 93 10201 A (MINNESOTA MINING & MFG)<br>* page 2, line 27 - line 38 *<br>* page 4, line 31 - line 41 *<br>* page 7, line 1 - page 9, line 27 * | 1-9 | |
| A | GB 2 115 431 A (VALLEYLAB INC)<br>* page 4, line 55 - page 5, line 14 *<br>* page 5, line 47 - page 7, line 13 *<br>* page 10, line 6 - line 28 *<br>* page 11, line 10 - line 19 * | 1-9 | TECHNICAL FIELDS SEARCHED (Int.Cl.6)<br><br>A61L<br>A61K<br>C09J |
| A | EP 0 184 470 A (MEDTRONIC INC)<br>* page 3, line 10 - line 19 *<br>* page 3, line 34 - page 5, line 7 * | 1-9 | |
| A | EP 0 730 874 A (SQUIBB BRISTOL MYERS CO)<br>* page 3, line 43 - line 55 *<br>* claims; examples * | 1-9 | |

The present search report has been drawn up for all claims

| Place of search | Date of completion of the search | Examiner |
|---|---|---|
| THE HAGUE | 29 April 1998 | Cousins-Van Steen, G |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

& : member of the same patent family, corresponding
  document

EPO FORM 1503 03.82 (P04C01)